# EUROPÄISCHE PATENTSCHRIFT

(11) **EP 0 835 895 B1**
(45) Veröffentlichungstag und Bekanntmachung des Hinweises auf die Patenterteilung: **03.12.2003**
(21) Anmeldenummer: 97116613.7
(22) Anmeldetag: 24.09.1997
(51) Int. Cl.: C08G 63/08, C08G 63/64, A61L 17/00

(54) **Chirugisches Nahtmaterial aus Triblockterpolymer, seine Verwendung in der Chirurgie und Verfahren zur Herstellung**
Surgical suture material from triblockterpolymer, its use in surgery and process for its preparation
Matériau de suture chirurgicale à partir de terpolymère tribloc, son emploi en chirurgie et son procédé de préparation

(30) Priorität: 08.10.1996 DE 19641335
(43) Veröffentlichungstag der Anmeldung: 15.04.1998
(73) Patentinhaber: DEUTSCHE INSTITUTE FÜR TEXTIL- UND FASERFORSCHUNG STUTTGART Stiftung des öffentlichen Rechts, 73770 Denkendorf (DE)
(72) Erfinder: Oberhoffner, Sven, Dr., 71384 Weinstadt-Benzach (DE); Planck, Heinrich, Prof. Dr.-Ing., 72622 Nürtingen (DE)
(74) Vertreter: Patentanwälte Ruff, Wilhelm, Beier, Dauster & Partner

(56) Entgegenhaltungen:
- EP-A- 0 098 394
- EP-A- 0 440 416
- EP-A- 0 441 322
- EP-A- 0 608 139
- EP-A- 0 636 639
- WO-A-94/11441
- DE-A- 4 300 420
- US-A- 5 431 679

## Beschreibung

Die vorliegende Erfindung betrifft ein chirurgisches Nahtmaterial aus einem Triblockterpolymer aus resorbierbarem synthetischem Polymer, seine Verwendung in chirurgischem Nahtmaterial und Verfahren zur Herstellung.

Für medizinische Produkte wie beispielsweise chirurgisches Nahtmaterial oder Implantate verwendbare resorbierbare synthetische Polymere umfassen klassische Homopolymere etwa aus Polyglykolsäure oder Polymilchsäure sowie deren Copolymere. Insbesondere bei Nahtmaterialen besitzen monofile Produkte gegenüber den geflochtenen Konstruktionen aus Multifilamenten den Vorteil, daß sie eine glatte, homogene Oberfläche aufweisen. Dies erleichtert den Fadenlauf und verringert das Auftreten von Kapillaritäten. Es müssen daher keine Beschichtungen zur Verbesserung des Fadenschlußes aufgebracht werden und die Durchzugkräfte durch das zu nähende Gewebe verringern sich aufgrund der glatten Oberfläche.

Ein Nachteil der bekannten Polymere für Nahtmaterial liegt in ihrer hohen Biegesteifigkeit, teilweise verbunden mit einer mangelnden Querfestigkeit, was zu einem schlechten Knüpfverhalten führt und die Verwendung für chirurgische Nähte einschränkt.

Die Entwicklung führte deshalb zum Einsatz von Blockcopolymeren, beispielsweise der Struktur AB, ABA oder ABAB, bei denen zumindest ein Block ein sogenanntes Weichsegment darstellt. Es ist bekannt, Weichsegmente durch Homo- oder Copolymerisation von Monomeren wie beispielsweise Trimethylencarbonat (1,3-Dioxan-2-on) TMC, ε-Caprolacton oder p-Dioxanon (1,4-Dioxan-2-on) herzustellen. Die Weichsegmente werden mit Hartsegmenten, deren Monomere typischerweise aus Glykolid und/oder Laktid ausgewählt sind, zu den entsprechenden Blockcopolymeren umgesetzt.

Unter den kommerziell vertriebenen langzeit-resorbierbaren Nahtmaterialien ist das im europäischen Patent EP 0098394 A1 der American Cyanamid Company offenbarte Blockcopolymer aus Glykolid und Trimethylencarbonat zu nennen.

Die internationale Anmeldung WO 94/11441 beschreibt ein Multiblockcopolymer mit hoher Dehnbarkeit und Schlagfestigkeit, bei dem Hartsegment auf Basis von Laktid und/oder Glykolid und Kautschuk-Weichsegment in separaten Phasen vorliegen.

Ein im europäischen Patent EP 0441322 A1 der ETHICON Inc. beschriebenes kristallines Copolymer aus Glykolid und ∈-Caprolacton stellt ein kurzzeit-resorbierbares Polymermaterial dar.

Im europäischen Patent EP 0626404 A2 der United States Surgical Corporation (USSC) sind absorbierbare Blockcopolymere aus Glykolid, p-Dioxanon und Trimethylencarbonat beansprucht, bei denen das Weichsegment nur aus p-Dioxanon und TMC gebildet ist.

In einem weiteren Patent der United States Surgical Corporation, US-Patent 5431679, ist ein Blockcopolymer beschrieben, das einen Block aus Glykolidestereinheiten und einen Block aus statistischen Copolymeren von 1,3-Dioxan-2-on und Caprolacton umfaßt.

Die Erfindung stellt sich die Aufgabe, ein chirurgisches Nahtmaterial aus einem resorbierbaren synthetischen Polymer in Form eines Triblockterpolymers zur Verfügung zu stellen, das ein gutes Abbau- und Resorptionsverhalten in vivo in Kombination mit guten mechanischen Eigenschaften besitzt, das einfach und kostengünstig herzustellen und auch einfach und zuverlässig für chirurgisches Nahtmaterial anzuwenden ist.

Diese Aufgabe wird gelöst durch ein chirurgisches Nahtmaterial ganz oder teilweise gebildet aus einem Triblockterpolymer mit einer Struktur ABA gebildet aus einem biologisch abbaubaren Hartsegment A und einem biologisch abbaubaren Weichsegment B, worin das Weichsegment B dihydroxyterminiert und an die beiden Hartsegmente A chemisch gebunden ist, dadurch gekennzeichnet, daß die Hartsegmentblöcke 50 bis 60 Gew.-% des Triblockterpolymers umfassen, das Weichsegment ein statistisches Terpolymer gebildet aus Trimethylencarbonat, ε-Caprolacton und Glykolid mit völlig amorpher Struktur ist, im Terpolymer des Weichsegments B Trimethylencarbonat in einem Anteil von 5 bis 70 Gew.-%, ε-Caprolacton in einem Anteil von 5 bis 70 Gew.-% und Glykolid in einem Anteil von 10 bis 70 Gew.-% enthalten ist, Trimethylencarbonat und ε-Caprolacton in einem Gewichtsverhältnis zwischen 80 : 20 und 20 : 80 vorhanden sind, und Glykolid als Monomer sowohl im Hartsegment A wie im Weichsegment B vorhanden ist.

Die vollständig amorphe Struktur des Weichsegments kann die Abbaubarkeit in vivo vorteilhaft beeinflussen. Das Abbauverhalten des Weichsegments nähert sich dem des Hartsegments dadurch an, daß es ebenfalls das schnell degradierbare Glykolid enthält. Durch seine vollständige Amorphität wurde überraschend ein relativ schnelles Degradationsverhalten gefunden, das wohl auf eine schnellere Diffusion der Hydrolyselösung, beziehungsweise der Körperflüssigkeiten zurückzuführen ist, als dies in kristallinen Bereichen der Fall sein kann. Auch in den strukturellen Merkmalen zeigt sich eine erhöhte Verträglichkeit von Weichsegment und Hartsegment. Es ergibt sich ein ausgeglichenes Resorptionsverhalten in vivo der Hart- und Weichsegmente im Triblockterpolymer.

Die Struktur des erfindungsgemäßen chirurgischen Nahtmaterials aus Triblockterpolymeren wirkt sich auch in vorteilhafter Weise auf die Eigenschaften daraus hergestellter Produkte aus. Als Beispiele sind günstige mechanische Eigenschaften wie gute Flexibilität, beispielsweise geringe Biegesteifigkeit, gutes Modulverhalten und gute Verknotungseigenschaften zu nennen, wie sie insbesondere bei Anwendungen im medizinischen Bereich, etwa bei chirurgischen Nähfäden, erwünscht sind.

Im erfindungsgemäßen Nahtmaterial aus Triblockterpolymer kann das Hartsegment A insbesondere ein Homopolymer sein. Beim Triblockterpolymer kann das Terpolymer im Weichsegment B ein Monomer enthalten, das im Hartsegment A enthalten ist. Im Triblockterpolymer umfasst der Anteil der Hartsegmentblöcke A 50 bis 60 Gew.-% des Triblockterpolymers und der Rest ist Weichsegment B.

Das Triblockterpolymer im Weichsegment B zeichnet sich dadurch aus, dass es aus Trimethylencarbonat, ε-Caprolacton und Glykolid gebildet ist. Insbesondere ist Trimethylencarbonat in einem Anteil von 5 bis 70 Gew.-%, ε-Caprolacton in einem Anteil von 5 bis 70 Gew.-% und Glykolid in einem Anteil von 10 bis 70 Gew.-% im erfindungsgemäßen Terpolymer enthalten. Die Gewichtsanteile der Komponenten Trimethylencarbonat, ε-Caprolacton und Glykolid sind so gewählt, dass sie zusammengenommen 100 Gew.-% des Terpolymers im Weichsegment B ausmachen. Erfindungsgemäß bevorzugt kann im Terpolymer Trimethylencarbonat insbesondere in einem Anteil von 10 bis 40 Gew.-%, ε-Caprolacton insbesondere in einem Anteil von 10 bis 40 Gew.-% und Glykolid insbesondere in einem Anteil von 30 bis 60 Gew.-% enthalten sein.

Im Terpolymer des Weichsegments B gemäß der vorliegenden Erfindung sind Trimethylencarbonat und ε-Caprolacton in einem Gewichtsverhältnis zwischen 80 : 20 und 20 : 80, insbesondere in einem Verhältnis zwischen 70 : 30 und 30 : 70 vorhanden. Bevorzugt können im WeichsegmentterpolymerTrimethylencarbonat und ε-Caprolacton in einem Gewichtsverhältnis von 50 : 50 vorhanden sein. In einer anderen Ausführungsform kann im Weichsegmentterpolymer ε-Caprolacton in einem höheren Anteil als Trimethylencarbonat vorhanden sein.

Das erfindungsgemäße Nahtmaterial aus einem Triblockterpolymer zeichnet sich insbesondere dadurch aus, das das sowohl im Hartsegment A wie im Weichsegment B vorhandene Monomer Glykolid ist. Bevorzugt kann das Terpolymer des Weichsegments B durch statistische Copolymerisation von Trimethylencarbonat, ε-Caprolacton und Glykolid hergestellt sein.

Im Triblockterpolymer ist mit Vorteil das Weichsegment B als Mittelblock zu beiden Seiten von Hartsegmentblöcken A umgeben. Das Hartsegment wird durch Polymerisationsreaktion an den OH-Gruppen an beiden Enden des Weichsegments angeknüpft. Der Aufbau des Hartsegments kann mit Vorteil durch Umsetzung des OH-terminierten Weichsegmentterpolymers mit Glykolidmonomeren vorgenommen sein. Ein Triblockterpolymerstrang gemäß der Erfindung umfaßt bevorzugt nur ein Weichsegment im Polymerstrang.

Es wurden Untersuchungen der physikalischen und physiologischen Eigenschaften des Triblockterpolymers vorgenommen, wie beispielsweise zur Mikrostruktur, zum Glasumwandlungsbereich, zum Schmelzverhalten und der inhärenten Viskosität sowie zur in vitro- und in vivo-Degradation und zum Resorptionsverhalten. Wenn nichts anderes angegeben ist erfolgen Viskositätsmessungen in Hexafluorisopropanol (HFIP) bei 30 °C und einer Konzentration von c = 0,8 g/dl. Messungen der Glastemperaturen (Tg), Schmelztemperaturen (Tm) und Schmelzenthalpien (Hm) werden mittels Differential Scanning Calorimetry (DSC) bei einer Scanrate von 10 °C/min vorgenommen. Die in vitro-Degradation wurde im Sörensen-Puffer bei pH 7,4 und einer Temperatur von 37 °C gemessen und als Retention in Bezug zur ursprünglichen Knotenreißkraft in Prozent angegeben.

Das Triblockterpolymer unterscheidet sich von den bisher beispielsweise zur Herstellung von chirurgischem Nahtmaterial üblichen Blockpolymeren durch die modifizierte Abfolge der Monomereinheiten in der Makromolekülkette. Dies beeinflußt auch die Wechselwirkungen zwischen den einzelnen Kettenmolekülen in einem gebildeten Filament. Wie den Fachleuten auf dem Gebiet der Fasertechnologie bekannt ist, hängen die physikalischen und mechanischen Eigenschaften einer Faser von der Orientierung und Struktur der Kettenmoleküle ab, insbesondere der Ausbildung amorpher und kristalliner Bereiche.

Bevorzugt kann das Triblockterpolymer eine inhärente Viskosität von 0,5 bis 1,5 dl/g, insbesondere von 0,7 bis 1,2 dl/g aufweisen. Das Triblockterpolymer kann ferner eine Glasumwandlungstemperatur zwischen -10 °C und 25 °C aufweisen. Bevorzugt kann das Weichsegment B im erfindungsgemäßen Triblockterpolymer eine Glasumwandlungstemperatur zwischen -30 °C und 10 °C aufweisen. Insbesondere zeichnet sich das Triblockterpolymer dadurch aus, daß es in seiner Struktur teilkristallin ist, wobei sich die Kristallinität auf das Hartsegment beschränkt. Die Schmelzenthalpie, ein Maß für die Kristallinität eines Polymers, beträgt für das Triblockterpolymer zwischen 15 bis 60 J/g, insbesondere 15 bis 50 J/g.

Das erfindungsgemäße Nahtmaterial aus dem resorbierbaren Triblockterpolymer zeichnet sich mit Vorteil durch eine beschleunigte Resorbierbarkeit in lebendem Gewebe aus. Seine Degradationsdauer in vitro kann 5 bis 30 Tage betragen (Sörensen-Puffer, 37 °C).

Es ist davon auszugehen, daß die Inkorporation eines dritten Monomers in zufälliger Verteilung in das Weichsegment die Tendenz zur Kristallisation des Weichsegments vermindert. Tatsächlich zeigten Untersuchungen mittels Differential Scanning Calorimetry (DSC), daß das Weichsegment B in der Struktur vollständig amorph ist. Eine Unterdrückung der Kristallisation im Weichsegment führt zu einer wünschenswerten Verbesserung der Flexibilität von Produkten, die aus dem Triblockterpolymer hergestellt sind. Meßwerte der mechanischen Eigenschaften von extrudierten Filamenten in zwei bevorzugten Ausführungsformen des erfindungsgemäßen Polymers sind in Tabelle 1 und Tabelle 2 angegeben.

Der Abbau des Polymers erfolgt im Körper eines Tieres oder eines Menschen durch Stoffwechselvorgänge. An der Umsetzung sind Körper- und Gewebeflüssigkeiten beteiligt. Durch Hydrolyse wird die Polymerkette in kleinere und leichter lösliche Fragmente gespalten. Die Bruchstücke werden ggf. unter Beteiligung enzymatischer Prozesse weiter abgebaut. Die Abbauprodukte werden durch das Stoffwechselsystem abtransportiert und wie andere Stoffwechselschlacken aus dem Organismus ausgeschieden. Für eine gute Verträglichkeit des resorbierbaren Nahtmaterials beim Patienten ist es wichtig, daß sich während des Abbauvorganges keine schädlichen Metaboliten bilden oder anreichern. Polyglykolsäure zeichnet sich insbesondere dadurch aus, daß bei ihrer Zersetzung in vivo keine toxischen Zerfallsprodukte gebildet werden. Die erfindungsgemäß als Comonomere verwendeten Trimethylencarbonat und Caprolacton sind ebenfalls durch gute Verträglichkeit und Vermeidung toxischer Reaktionen gekennzeichnet.

Im Vergleich zu Glykolid weisen Trimethylencarbonat und Caprolacton viel längere Degradationszeiten auf. Daraus kann ein stark differierendes Resorptionsverhalten von Hartsegment (z. B. Glykolid) und Weichsegment (z. B. TMC/Caprolacton-Copolymer gemäß dem Stand der Technik) resultieren. Unverträgliche Polymere bzw. Polymersegmente neigen zur Phasentrennung, die sich - wie aus Tabelle 2 ersichtlich ist - überraschend in einer Verschlechterung der mechanischen Festigkeiten bemerkbar macht.

Durch das Einpolymerisieren von Glykolid in das Weichsegment kann die Verträglichkeit zwischen Hartsegment und Weichsegment erhöht werden. Dies kann sich einerseits vorteilhaft auf die für die Praxis wichtigen mechanischen Eigenschaften des Polymers auswirken. Ferner kann sich daraus eine einheitlichere Degradation und Resorption der Weich- und Hartsegmentanteile der Blockcopolymere im lebenden Organismus ergeben.

Das Degradationsverhalten des erfindungsgemäßen Nahtmaterials aus Triblockterpolymer kann durch Variation des Gesamtglykolidanteils im Polymer verändert werden. Ferner kann das Degradationsverhalten des Triblockterpolymers durch Variation des Anteils an Weichsegment B im Triblockterpolymer verändert werden (siehe Tabelle 3). Ein weiterer Einflußfaktor, durch dessen Variation das Abbauverhalten des Polymers verändert werden kann, ist die Intensität und Dauer einer etwaigen γ-Bestrahlung. Die Behandlung mit γ-Strahlen kann mit einem teilweisen Molekulargewichtsabbau verbunden sein, der sich in verkürzten Abbauzeiten äußert. Auf diese Weise ist es möglich, die Eigenschaften des Triblockterpolymers nach den für den Anwendungsfall vorteilhaften Erfordernissen anzupassen. In einer möglichen Ausführungsform der Erfindung kann eine mit Hilfe von γ-Strahlen durchgeführte Sterilisation gleichzeitig zur Steuerung des Degradationsverhaltens der aus dem Polymer hergestellten chirurgischen Nahtmaterialien dienen.

Es wurde gefunden, daß das Triblockterpolymer mit einer Struktur ABA gebildet aus einem Hartsegment A aus biologisch abbaubarem Monomer und einem Weichsegment B aus biologisch abbaubarem Monomer, worin das Weichsegment ein statistisches dihydroxyterminiertes Terpolymer mit amorpher Struktur ist, als resorbierbares Polymer zur Herstellung eines chirurgischen Nahtmaterials geeignet ist. Mit Vorteil kann das für medizinische Anwendung vorgesehene Produkt ganz oder teilweise aus dem Polymer gebildet sein.

Es wurde überraschend festgestellt, daß aus dem Blockpolymer chirurgische Nahtmaterialien hergestellt werden können, insbesondere Monofilamente für Nahtmaterial, die für chirurgisches Material erforderliche sehr gute Eigenschaften aufweisen, besonders bezüglich physikalischer Eigenschaften und praktischer Handhabung.

Wie aus der obigen Beschreibung der Eigenschaften des polymerischen Nahtmaterials gemäß der Erfindung ersichtlich ist, zeichnet es sich besonders durch seine biologische Abbaubarkeit und sein günstiges Abbauverhalten und die guten mechanischen Eigenschaften, insbesondere seine Flexibilität, für Anwendungen im medizinischen Bereich aus.

Mit Vorteil ist chirurgisches Nahtmaterial aus resorbierbarem synthetischem Polymer ausgebildet als monofiles Filament aus dem Triblockterpolymer aus Glykolid, Trimethylencarbonat und Caprolacton zur Verwendung zum Wundverschluß mit beschleunigter Resorption geeignet. Die oben genannten vorteilhaften mechanischen Eigenschaften von monofilen Nähfäden aus Triblockterpolymer erlauben eine einfache Handhabung des Nahtmaterials während des Vernähens von Gewebe in einem tierischen oder menschlichen Körper, z. B. beim Fixieren von Organen, Schließen von Rissen im Körpergewebe oder Schließen von chirurgischen Schnitten. Insbesondere durch die Ausbildung eines Monofilaments mit glatterer Fadenoberfläche als ein multifiler Nähfaden kann das zu behandelnde Gewebe vor Schädigung durch die Nahtlegung bewahrt werden. Dadurch wird die Gefahr von für den Patienten belastenden Nebenwirkungen, wie z. B. verzögerte Heilung und Gewebegranulombildung eingeschränkt. Eine gute Knüpfbarkeit und Knotenfestigkeit in Verbindung mit einer hohen Anfangszugfestigkeit und Flexibilität erlauben eine zuverlässige Fixierung und Stabilisierung der verbundenen Wundränder während der ersten Tage nach dem chirurgischen Eingriff. Insbesondere kann während dieser ersten Tage sich neubildendes körpereigenes Gewebe zuverlässig zur natürlichen Wundheilung dienen, da die Gefahr eines Auseinanderreissens der Wundränder bei Bewegung des Patienten durch die sichere Fixierung verringert ist.

Die vorliegende Erfindung stellt auch ein Verfahren zur Verfügung zur Herstellung eines chirurgischen Nahtmaterials ganz oder teilweise gebildet aus einem Triblockterpolymer mit einer Struktur ABA gebildet aus einem biologisch abbaubaren Hartsegment A und einem biologisch abbaubaren Weichsegment B, worin das Weichsegment dihydroxyterminiert ist und an die beiden Hartsegmente A chemisch gebunden wird, das dadurch gekennzeichnet ist, daß das Triblockterpolymer durch chemisches Umsetzen des Hartsegmentmonomers mit Hydroxyendgruppen des Weichsegments B, das ein statistisches Terpolymer aus Trimethylencarbonat, ε-Caprolacton und Glykolid mit völlig amorpher Struktur ist, gebildet wird, so dass ein Triblockterpolymer mit einem Hartsegmentanteil von 50 bis 60 Gew.-% ausgebildet wird, wobei zuvor das Weichsegment durch statistische Copolymerisation von Trimethylencarbonat, ε-Caprolacton und Glykolid, bei einem Gewichtsanteil von Trimethylencarbonat von 5 bis 70 Gew.-%, bevorzugt 10 bis 40 %, ε-Caprolacton von 5 bis 70 Gew.-%, bevorzugt 10 bis 40 %, und Glykolid von 10 bis 70 Gew.-%, bevorzugt 30 bis 60 %, hergestellt und zu Filamenten versponnen wird.

Die Gewichtsanteile der Komponenten Trimethylencarbonat, ε-Caprolacton und Glykolid werden dabei so gewählt, daß sie zusammengenommen 100 Gew.-% des Terpolymers im Weich segment B ausmachen.

Der Monomerenmischung zur Herstellung des Weichsegments kann ein geeigneter Katalysator, z. B. Zinnoctoat, sowie ein bifunktioneller Initiator, z. B. Diethylenglykol, in der üblichen erforderlichen Menge zugesetzt werden. Die Umsetzung wird als Schmelzpolymerisation bei Temperaturen über 150 °C in einem geeigneten Reaktor durchgeführt, der heizbar und mit einer Rührvorrichtung versehen ist. Insbesondere muß dieser Polymerisationsreaktor so konzipiert sein, daß die entstehenden hochviskosen Schmelzen homogenisiert, die geforderten Temperaturbereiche eingehalten werden können und das Rohpolymerisat aus dem Reaktor weitgehend vollständig abgelassen werden kann.

Die Terpolymerisierungsreaktion kann nach üblichen den Fachleuten bekannten Verfahrenweisen zur Herstellung von statistischen Copolymeren durchgeführt werden. Bevorzugt kann die Reaktionsmischung unter stetiger Durchmischung erhitzt werden, insbesondere auf eine Temperatur von 190 bis 210 °C, bevorzugt von 205 °C. Für die Dauer der Umsetzung wird ein Überdruck von 1 bis 2 bar Argon, bevorzugt 1,5 bar Argon angelegt. Während einer Reaktionsdauer von 2 bis 6 Stunden, bevorzugt 5 h, können sich die vorgelegten Monomeren zu einem statistischen Terpolymer umsetzen. Mit Vorteil zeichnet sich das Verfahren dadurch aus, daß die Umsatzrate bei der Weichsegmentpolymerisation über 95 % beträgt.

In einer Ausführungsform des erfindungsgemäßen Verfahrens kann das Weichsegment nach der Polymerisation isoliert werden und nach erneutem Aufschmelzen mit Glykolid zum Triblockterpolymer umgesetzt werden. Dazu wird nach Beendigung der Umsetzung das rohe Terpolymer des Weichsegments B als Schmelze ausgetragen und nach Abkühlen zerkleinert.

Die Umsetzung des Weichsegmentterpolymers mit Glykolidmonomer zum Triblockterpolymer erfolgt in bekannter Weise als Schmelzpolymerisation in einem geeigneten Polymerisationsreaktor wie es oben für die Herstellung des Weichsegments beschrieben ist. Es kann wiederum ein geeigneter Katalysator, z. B. Zinnoctoat, sowie ein bifunktioneller Initiator, z. B. Diethylengylkol, in der üblichen erforderlichen Menge zugesetzt werden. Bevorzugt wird die Reaktionsmischung über einen Zeitraum von 0,5 bis 1 Stunde auf eine Temperatur von 200 bis 250 °C, bevorzugt von 220 bis 240 °C erhitzt. Die Zuschaltung einer Rühreinrichtung erfolgt bevorzugt nach Erreichen einer Temperatur von etwa 130 °C. Für die Dauer der Umsetzung wird ein Überdruck von 1 bis 2 bar Argon, bevorzugt 1,5 bar Argon angelegt. Während einer Reaktionsdauer von 1 bis 3 Stunden bildet sich das Triblockterpolymer mit Hartund Weichsegmenten der Struktur ABA. Anschließend wird das Polymer aus dem Reaktor abgelassen und nach Erkalten in üblicher Weise zerkleinert und getrocknet.

In einer weiteren Ausführungsform des erfindungsgemäßen Verfahrens kann das Weichsegment nach der Polymerisation direkt in situ mit Glykolid zum Triblockterpolymer umgesetzt werden. Die In-situ-Polymerisation des Triblockterpolymers gemäß der Erfindung erfolgt als Schmelzpolymerisation in einem Polymerisationsreaktor wie es für die oben genannten Polymerisationsreaktionen beschrieben ist. Zunächst werden die Monomere Glykolid, 1,3-Dioxan-2-on und Caprolacton in den für die Bildung des Weichsegments erforderlichen Mengen zusammen mit dem benötigten Katalysator und Initiator in den Reaktor gegeben. Unter Rühren wird die Mischung bei einem Argon-Überdruck von 1 bis 2 bar während etwa 30 min auf eine Temperatur von 200 bis 210 °C erhitzt und während 4 bis 6 h unter diesen Bedingungen umgesetzt. Dann wird zur Bildung des Triblockterpolymers eine erforderliche Menge des Hartsegmentmonomers Glykolid als Schmelze zugegeben. Die Umsetzung zur Hartsegmentbildung erfolgt unter Argongegenstrom und unter heftigem Rühren. Dabei wird die Temperatur innerhalb von weniger als 15 min auf etwa 230 °C erhöht, danach auf etwa 220 °C verringert und diese Bedingungen zur Vervollständigung der Umsetzung etwa 1 bis 2 Stunden aufrechterhalten. Das fertige Triblockterpolymer wird abgelassen und nach Erkalten in üblicher Weise zerkleinert und getrocknet.

Aus den Triblockterpolymeren können durch übliche Schmelzspinnprozesse Produkte zur Verwendung als resorbierbare chirurgische Nahtmaterialien hergestellt werden. In einer Ausführungsform des Verfahrens kann das Triblockterpolymer zu Filamenten extrudiert werden. In einer bevorzugten Ausführungsform kann das Triblockterpolymer in einem Schmelzspinnverfahren, z. B. einem Einschneckenextruder oder einem Doppelschneckenextruder, durch geeignete Spinndüsen zu Monofilamenten extrudiert werden. Beim Schmelzspinnen liegt die Düsentemperatur insbesondere um bis zu 30 °C über dem Schmelzpunkt des verarbeiteten Polymers.

Mit Vorteil kann das gebildete Filament zur Verfestigung in ein Kühlbad, das Wasser oder eine übliche organische Flüssigkeit wie beispielsweise Glyzerin enthält, extrudiert werden. Die Kühlbadtemperatur kann im Bereich von 2 bis 60 °C, insbesondere 2 bis 50 °C liegen. Bevorzugt ist eine Extrusion des Filaments in Wasser bei Raumtemperatur. Der Abstand zwischen Spinndüse und Kühlbad liegt zwischen 0,5 und 30 cm, bevorzugt zwischen 1 und 10 cm.

Um die erforderlichen mechanischen Eigenschaften zu erhalten, kann das extrudierte Filament zur Orientierung der Molekülketten verstreckt werden. Der verfestigte Spinnfaden kann entweder direkt oder nach Aufspulen in einem gesonderten Schritt nach gängigen Methoden verstreckt werden. Dabei kann die Verstreckung wahlweise in beheizten flüssigen Medien wie beispielsweise Wasser- oder Glyzerinbädern oder über Verstrecköfen und -schienen durchgeführt werden. Mit Vorteil kann es mit einem Verstreckverhältnis von 1 : 4 bis 1 : 10 verstreckt werden.

Um einen andauernden Erhalt der Orientierung, der mechanischen Eigenschaften sowie der Dimensionsstabilität der Filamente zu sichern, kann das verstreckte Polymermaterial durch Tempern fixiert werden. Das Fixieren erfolgt bei Temperaturen im Bereich zwischen 50 °C und 150 °C, bevorzugt bei 70 bis 130 °C. Die Dauer des Thermofixierverfahrens liegt zwischen einer und 20 Stunden. Das Tempern kann mit oder ohne Schrumpfen des Filaments vorgenommen werden. Besonders bevorzugt ist es, Verstrecken und Thermofixieren unmittelbar anschließend an die Extrusion, insbesondere in einem kombinierten Verfahren durchzuführen. Mit Vorteil kann dazu eine entsprechende Apparatur von kombinierten geeigneten Vorrichtungen verwendet werden. In einer bevorzugten Ausführungsform der Erfindung können monofile oder multifile Produkte aus dem Triblockterpolymer zur Erzielung einer Dimensionsstabilität über einen Zeitraum von 1 bis 20 Stunden, mit oder ohne Schrumpf, einer Temperatur von 50 bis 150 °C ausgesetzt werden.

Die Durchmesser der auf diese Weise hergestellte Monofilamente können im üblichen Bereich von 0,001 bis 1,2 mm liegen. Mit Vorteil sind diese Monofilamente gemäß der Erfindung ferner durch die oben genannten mechanischen Eigenschaften gekennzeichnet.

Als Beispiele für die Anwendung in medizinischen Produkten sind zu nennen durch Verspinnen hergestellte Filamente, die zur Verwendung als im Organismus des Patienten resorbierbares chirurgisches Nahtmaterial direkt in Form von Monofilamenten oder in Form von multifilen Fadenkonstruktionen eingesetzt werden.

Die Polymere und das daraus hergestellte Nahtmaterial gemäß der vorliegenden Erfindung können gefärbt oder ungefärbt sein. Zum Einfärben können für resorbierbare medizinische Produkte von der amerikanischen FDA (Food and Drug Administration) zugelassene Farbstoffe verwendet werden wie beispielsweise D&C Green Nr. 6, D&C Violet Nr. 2 und andere.

Erfindungsgemäß hergestellte Triblockterpolymer-Filamente können nach üblichen Methoden zu chirurgischem Nahtmaterial verarbeitet, z. B. auf geeignete Längen zugeschnitten werden. Insbesondere kann das Polymermaterial in geeigneter Weise sterilisiert werden. Ein zweckmäßiges Sterilisierverfahren kann aus üblichen physikalischen oder chemischen Methoden zum Inaktivieren von Mikroorganismen ausgewählt oder eine Kombination solcher Methoden sein. Ein mögliches Sterilisierungsverfahren umfaßt die Behandlung mit γ-Strahlung. Bevorzugt kann eine Sterilisierung des erfindungsgemäßen Polymermaterials für medizinische Produkte unter Verwendung von Ethylenoxid vorgenommen werden.

Mit Vorteil kann aus dem Polymer hergestelltes chirurgische Nahtmaterial in zweckmäßiger Länge zugeschnitten gebrauchsfertig in geeigneter Weise verpackt vorliegen. In einer bevorzugten Ausführungsform können die erfindungsgemäßen Nähfäden mit chirurgischen Nadeln versehen vorliegen.

Wegen der hydrolytischen Zersetzbarkeit des erfindungsgemäßen Nahtmaterials sind die medizinischen Produkte bei ihrer Lagerung vor Feuchtigkeit und erhöhten Temperaturen zu schützen, damit die Festigkeitseigenschaften bis zur Verwendung voll erhalten bleiben. Mit Vorteil können gemäß der Erfindung hergestellte medizinischen Nähfäden in gebrauchsbereitem Zustand getrocknet und in geeigneter Weise verpackt werden. Zweckmäßigerweise kann dies durch eine vor Feuchtigkeit schützende Verpackung geschehen, insbesondere einer Verpackung aus feuchtigkeitsundurchlässigem Folienmaterial, bevorzugt einer Vakuumverpackung. Ferner durch Auswahl eines trockenen kühlen Lagerortes.

Die Polymere und daraus hergestellte Produkte zeichnen sich insbesondere durch die nachfolgenden physikalischen Eigenschaften aus: es handelt sich um semikristallines und damit bei Raumtemperatur festes Triblockpolymer, das eine feste Konsistenz aufweist. Das Triblockpolymer weist einen Schmelzpunkt auf, der über 120 °C liegt. Zwischen Hart- und Weichsegment liegt keine Phasenseparation vor. Dies geht aus der Glasumandlungstemperatur hervor, die für das Terpolymer gemäß der Erfindung bei -10 bis +30 °C, insbesondere bei 0°C bis +15 °C liegt. Bei mehreren Phasen wären getrennt detektierbare Glasumwandlungspunkte vorhanden.

Die inhärente Viskosität des Triblockterpolymers liegt mit Vorteil über 0,7 dl/g in HFIP (c = 0,8 g/l bei 30 °C). Für praktisch einsetzbare Polymere kann die inhärente Viskosität bis zu 2,0 dl/g betragen.

Damit im Polymer der Restmonomerengehalt gering ist und gleichzeitig eine hohe Umsatzrate erreicht wird, kann die Weichsegmentpolymerisation in der Schmelze bei mehr als 150 °C, vorzugsweise oberhalb von 170 °C, bis zu 235 °C erfolgen.

Im Falle, wo das Polymer in Fäden umgewandelt und insbesondere verstreckt ist, verändert sich während der Degradationsdauer die Knotenreißfestigkeit. Sie liegt nach 7 Tagen zwischen 30 und 80 %, bevorzugt zwischen 50 und 70 % des ursprünglichen Wertes. Nach 14 Tagen beträgt die Knotenreißfestigkeit noch zwischen 5 bis 50 %, insbesondere 20 bis 40 % des ursprünglichen Wertes, wie aus Messungen in Sörensen-Puffer bei pH 7,4 und 37 °C ersichtlich ist.

Ferner liegt beim verstreckten Polymermaterial gemäß der Erfindung, insbesondere bei verstreckten Fäden, die Dehnung zwischen 15 und 60 %, bevorzugt zwischen 25 und 45 %. Die linearen Reißkräfte liegen zwischen 300 bis 1000 N/mm², insbesondere über 400 N/mm². Die Knotenreißkraft liegt zwischen 250 bis 800 N/mm², vorzugsweise oberhalb von 350 N/mm².

Für das erfindungsgemäße Nahtmaterial liegt der Elastizitätsmodul zwischen 500 bis 3000 N/mm², vorzugsweise unterhalb von 1800 N/mm². Im Falle von Multifilenfäden können Werte für den Elastizitätsmodul bis 7000 N/mm², vorzugsweise weniger als 5000 N/mm² betragen.

Weitere Merkmale und Einzelheiten der Erfindung ergeben sich aus der nachfolgenden Beschreibung von bevorzugten Ausführungsformen in Form von Beispielen. Dabei können die einzelnen Merkmale jeweils für sich alleine oder zu mehreren in Kombination miteinander verwirklicht sein. Die Beispiele dienen lediglich der Erläuterung der vorliegenden Erfindung.

### Beispiel 1

### Dihydroxyterminiertes Weichsegment der Zusammensetzung G/TMC/CL = 30/35/35

In einen Reaktor werden 350 g 1,3-Dioxan-2-on (TMC), 350 g Caprolacton (CL) und 300 g Glykolid (G) gegeben zusammen mit 0,2 g Zinnoctoat (Lösung in Diethylether) und 1 g Diethylenglykol. Der Ether wird danach im Hochvakuum bei 50 °C abgezogen. Nach einer Stunde wird ein Überdruck von 1,5 bar Argon angelegt und der Reaktor unter Rühren auf 205 °C erhitzt. Diese Temperatur wird 5 h aufrechterhalten. Danach wird das Polymer abgelassen und analysiert. Die inhärente Viskosität beträgt 0,648 dl/g, die Glastemperatur liegt bei - 27,5 °C.

### Beispiel 2

### Dihydroxyterminiertes Weichsegment der Zusammensetzung G/TMC/CL = 40/30/30

In einen Reaktor werden 300 g 1,3-Dioxan-2-on, 300 g Caprolacton und 400 g Glykolid gegeben zusammen mit 0,2 g Zinnoctoat (Lösung in Diethylether) und 1 g Diethylenglykol. Die Umsetzung zum Polymer erfolgt analog zum Beispiel 1. Die inhärente Viskosität beträgt 0,937 dl/g, die Glastemperatur liegt bei -19,8 °C.

### Beispiel 3

### Dihydroxyterminiertes Weichsegment der Zusammensetzung G/TMC/CL = 50/25/25

In einen Reaktor werden 250 g 1,3-Dioxan-2-on, 250 g Caprolacton und 500 g Glykolid gegeben zusammen mit 0,2 g Zinnoctoat (Lösung in Diethylether) und 1 g Diethylenglykol gegeben. Die Umsetzung erfolgt analog wie in Beispiel 1. Die inhärente Viskosität beträgt 0,813 dl/g, die Glastemperatur liegt bei -9,3 °C.

### Beispiel 4

### Triblockterpolymer der Zusammensetzung G/TMC/CL = 72/14/14 mit 40 Gew.-% des Weichsegments aus Beispiel 1

In einen Reaktor werden 600 g Glykolid und 400 g des Weichsegments aus Beispiel 1 gegeben zusammen mit 0,1 g Zinnoctoat (Lösung in Diethylether). Der Ether wird im Hochvakuum bei 50 °C abgezogen. Nach Anlegen eines Überdruckes von 1,5 bar Argon wird der Reaktor über einen Zeitraum von 40 min auf 240 °C erhitzt. Die Zuschaltung eines Rührers erfolgt nach Erreichen einer Temperatur von 130 °C. Die Temperatur von 240 °C wird 70 min aufrechterhalten und das Polymer anschließend abgelassen. Die inhärente Viskosität des ABA-Triblockterpolymers beträgt 0,75 dl/g, die Glastemperatur liegt bei 9,5 °C und der Schmelzpunkt bei 182,3 °C.

### Beispiel 5

### Triblockterpolymer der Zusammensetzung G/TMC/CL = 73/13,5/13,5 mit 45 Gew.-% des Weichsegments aus Beispiel 2

Es werden 550 g Glykolid und 450 g des Weichsegments aus Beispiel 2 ohne zusätzliche Katalysatorzugabe in den Reaktor gegeben und 16 Stunden bei 60 °C im Hochvakuum getrocknet. Nach Anlegen eines Überdruckes von 1,5 bar Argon wird der Reaktor über einen Zeitraum von 35 min auf 235 °C erhitzt, wobei der Rührer nach Erreichen einer Temperatur von 130 °C zugeschaltet wird. Die Temperatur von 235 °C wird 60 min aufrechterhalten und das Polymer anschließend abgelassen.

Die inhärente Viskosität des ABA-Triblockterpolymers beträgt 1,01 dl/g, die Glastemperatur liegt bei 9,8 °C und der Schmelzpunkt bei 180,1 °C.

### Beispiel 6 (Vergleichsbeispiel)

### Triblockterpolymer der Zusammensetzung G/TMC/CL = 73/13,5/13,5 mit 54 Gew.-% des Weichsegments aus Beispiel 3

Es werden 460 g Glykolid und 540 g des Weichsegments aus Beispiel 3 zusammen mit 0,05 g Zinnoctoat (Lösung in Diethylether) in den Reaktor gegeben und 16 Stunden bei 50 °C im Hochvakuum getrocknet. Nach Anlegen eines Überdruckes von 1,5 bar Argon wird der Reaktor über einen Zeitraum von 45 min auf 230 °C erhitzt, wobei der Rührer nach Erreichen einer Temperatur von 130 °C zugeschaltet wird. Die Temperatur wird nach 10 min auf 220 °C verringert und auf diesem Niveau 100 min aufrechterhalten. Anschließend wird das Polymer abgelassen.

Die inhärente Viskosität des ABA-Triblockterpolymers beträgt 0,813 dl/g, die Glastemperatur liegt bei 9,9 °C und der Schmelzpunkt bei 164,5 °C.

### Beispiel 7

### In-situ-Polymerisation eines Triblockterpolymers der Zusammensetzung G/TMC/CL = 73/13,5/13,5 mit 45 Gew.-% eines Weichsegments der Zusammensetzung G/TMC/CL = 40/30/30

In der ersten Stufe werden 360 Glykolid, 270 g 1,3-Dioxan-2-on und 270 g Caprolacton zusammen mit 0,9 g Diethylenglykol und 0,2 g Zinnoctoat (Lösung in Diethylether) in den Reaktor gegeben. Nach 16 h Trocknung bei 50 °C im Hochvakuum wird ein Überdruck von 1,5 bar Argon angelegt und das Reaktionsgemisch unter Rühren über einen Zeitraum von 30 min auf 205 °C erhitzt. Diese Temperatur wird 5 h beibehalten. In Stufe 2 erfolgt die Zugabe von 1100 g aufgeschmolzenem Glykolid zur Bildung der Hartsegmente unter Argongegenstrom bei heftigem Rühren. Gleichzeitig wird die Temperatur für 10 min auf 230 °C erhöht, anschließend auf 220 °C gesenkt und dort für weitere 90 min gehalten.

Das Polymer besitzt eine inhärente Viskosität von 1,02 dl/g, die Glastemperatur liegt bei 2,1 °C und der Schmelzpunkt bei 191,2 °C. Eine vor der Glykolidzugabe entnommene Probe des Weichsegments weist eine inhärente Viskosität von 1,081 dl/g auf, die Glastemperatur liegt bei -20,1 °C.

### Beispiel 8 (Vergleichsbeispiel)

### In-situ-Polymerisation eines Triblockterpolymers der Zusammensetzung G/TMC/CL = 73/13,5/13,5 mit 54 Gew.-% eines Weichsegments der Zusammensetzung G/TMC/CL = 50/25/25

In der ersten Stufe werden 540 g Glykolid, 270 g 1,3-Dioxan-2-on und 270 g Caprolacton zusammen mit 1,08 g Diethylenglykol und 0,216 g Zinnoctoat (Lösung in Diethylether) in den Reaktor gegeben. Nach 16 h Trocknung bei 50 °C im Hochvakuum wird ein Überdruck von 1,5 bar Argon angelegt und das Reaktionsgemisch unter Rühren über einen Zeitraum von 30 min auf 205 °C erhitzt. Diese Temperatur wird 5 h beibehalten. In der Stufe 2 erfolgt die Zugabe von 1100 g aufgeschmolzenem Glykolid zur Bildung der Hartsegmente unter Argongegenstrom bei heftigem Rühren. Gleichzeitig wird die Temperatur für 10 min auf 230 °C erhöht, anschließend auf 220 °C gesenkt und dort für weitere 80 min gehalten.

Das Polymer besitzt eine inhärente Viskosität von 0,99 dl/g, seine Glastemperatur liegt bei 10,4 °C und der Schmelzpunkt bei 183,6 °C.

### Beispiel 9

### Extrusion des ABA-Triblockterpolymers zum Monofilament

Das Triblockterpolymer aus Beispiel 7 wird mit einem Doppelschneckenextruder bei einer Schneckendrehzahl von 21 Upm aufgeschmolzen und zu Monofilamenten versponnen. Das L/D-Verhältnis der Düsenkapillare beträgt 24 : 1. Die Düsentemperatur liegt bei 205 °C, also über der Schmelztemperatur des Polymers (191 °C). Der extrudierte Polymerstrang wird zur Verfestigung durch ein Kühlbad mit Wasser von 20 °C durchgezogen. Der Abstand zwischen Düse und Bad beträgt 6 cm. Der feste monofile Faden wird aufgespult. Zum Verstrecken wird das Monofilament anschließend über beheizte Verstreckschienen geführt. Dabei ist die erste Schiene auf 30 °C erhitzt, die zweite auf 60 °C. Das Verstreckverhältnis beträgt beim ersten Durchgang 6,8 : 1, beim zweiten 1,32 : 1, woraus sich ein Gesamtverstreckverhältnis von 9,0 : 1 ergibt. Zur Erzielung einer ausreichenden Dimensionsstabilität werden die verstreckten Fäden dann in einem weiteren Verfahrensschritt bei einer Temperatur von 80 °C 5 Stunden lang getempert. Der thermofixierte Faden wird zur Verwendung als chirurgischer Nähfaden mit einer Nadel versehen, verpackt und sterilisiert. Die mechanischen Eigenschaften des Fadens sind in Tabelle 1 angegeben.

### Beispiel 10 (Vergleichsbeispiel)

### Extrusion des ABA-Triblockterpolymers zum Monofilament

Das Triblockterpolymer aus Beispiel 8 wird entsprechend wie in Beispiel 9 zum Monofilament versponnen.

Die Verfahrensbedingungen für Extrusion, Verstrecken und Tempern sowie die mechanischen Eigenschaften der Monofilamente aus Beispiel 9 und 10 sind in der folgenden Tabelle 1 angegeben.

**Tabelle 1**

| | Beispiel 9 | Beispiel 10 |
|---|---|---|
| Extrusion | | |
| Polymer | aus Beisp. 7 | aus Beisp. 8 |
| Schneckendrehzahl (Upm) | 21 | 22 |
| Düsentemperatur (°C) | 205 | 185 |
| Düsendruck (bar) | 45 | 72 |
| Kapillardurchmesser (mm) | 1,8 | 1,8 |
| Abstand Düse-Bad (cm) | 6 | 4 |
| Badmedium | Wasser | Wasser |
| Badtemperatur (°C) | 20 | 20 |
| Abzuggeschw. (m/min) | 10,0 | 8,0 |

| Verstrecken | | |
|---|---|---|
| Verstreckverhältnis 1 | 6,8 : 1 | 6 : 1 |
| Temperatur Schiene 1 (°C) | 30 | 40 |
| Verstreckverhältnis 2 | 1,32 : 1 | 1,1 : 1 |
| Temperatur Ofen 2 (°C) | 60 | 80 |
| Gesamtverstreckung | 9,0 : 1 | 6,6 : 1 |
| Filamentdurchmesser (mm) | 0,352 | 0,472 |
| Linearreißkraft (N) | 49,5 | 73,5 |
| Knotenreißkraft (N) | 34,6 | 49,3 |
| E-Modul (N/mm²) | 848 | 645 |
| Dehnung (%) | 29,2 | 30,3 |

| Tempern (kein Schrumpf) | | |
|---|---|---|
| Temperdauer (h) | 5 | 10 |
| Tempertemperatur (°C) | 80 | 100 |
| Filamentdurchmesser (mm) | 0,349 | 0,472 |
| Linearreißkraft (N) | 54,4 | 75,9 |
| Knotenreißkraft (N) | 35,9 | 53,4 |
| E-Modul (N/mm²) | 1317,8 | 798,5 |
| Dehnung (%) | 24,9 | 32,7 |

### Beispiel 11 (Vergleichsbeispiel)

### In-situ-Polymerisation eines Triblockterpolymers der Zusammensetzung G/TMC/CL = 72/14/14 mit 38 Gew.-% eines Weichsegments der Zusammensetzung G/TMC/CL = 26/37/37

In der ersten Stufe werden 197,6 g Glykolid, 281,2 g 1,3-Dioxan-2-on und 281,2 g Caprolacton zusammen mit 760 mg Diethylenglykol und 150 mg Zinnoctoat (Lösung in Diethylether) in den Reaktor gegeben und entsprechend Beispiel 7 polymerisiert mit dem Unterschied, daß in der 2. Stufe 1240 g Glykolid zugegeben werden und die Reaktionstemperatur anschließend von 205 °C auf 225 °C erhöht und für weitere 90 min gehalten wird.

Die inhärente Viskosität des Weichsegments vor der Glykolidzugabe beträgt 0,994 dl/g, dessen Glaspunkt liegt bei -26,9 °C.
Das Triblockterpolymer besitzt eine inhärente Viskosität von 0,883 dl/g und einen Schmelzpunkt von 215,3 °C. Das Auftreten zweier getrennter Glastemperaturen von -16,8 °C und 28,3 °C ist ein Indiz für eine zumindest partielle Unverträglichkeit zwischen Hart- und Weichsegment.

### Beispiel 12

### In-situ-Polymerisation eines Triblockterpolymers der Zusammensetzung G/TMC/CL = 72/14/14 mit 40 Gew.-% eines Weichsegments der Zusammensetzung G/TMC/CL = 30/35/35

In der ersten Stufe werden 240,0 g Glykolid, 280,0 g 1,3-Dioxan-2-on und 280,0 g Caprolacton zusammen mit 800 mg Diethylenglykol und 160 mg Zinnoctoat (Lösung in Diethylether) in analoger Reaktionsführung zu Beispiel 10 polymerisiert und in der 2. Stufe mit 1200,0 g Glykolid zum Triblockterpolymer umgesetzt.

Die inhärente Viskosität des Weichsegments vor der Glykolidzugabe beträgt 1,112 dl/g, dessen Glaspunkt liegt bei -21,8 °C.
Das Triblockterpolymer besitzt eine inhärente Viskosität von 0,91 dl/g und einen Schmelzpunkt von 209,4 °C. Im DSC ist ein einzelner, jedoch verbreiterter Glasübergang von 11,3 °C detektierbar.

### Beispiel 13

### In-situ-Polymerisation eines Triblockterpolymers der Zusammensetzung G/TMC/CL = 72/14/14 mit 43 Gew.-% eines Weichsegments der Zusammensetzung G/TMC/CL = 35/32,5/32,5

In der ersten Stufe werden 301,0 g Glykolid, 279,5 g 1,3-Dioxan-2-on und 279,5 g Caprolacton zusammen mit 900 mg Diethylenglykol und 180 mg Zinnoctoat (Lösung in Diethylether) in analoger Reaktionsführung zu Beispiel 10 polymerisiert und in der 2. Stufe mit 1140,0 g Glykolid zum Triblockterpolymer umgesetzt.

Die inhärente Viskosität des Weichsegments vor der Glykolidzugabe beträgt 1,144 dl/g, der Glaspunkt liegt bei -19,1 °C. Das Triblockterpolymer besitzt eine inhärente Viskosität von 1,036 dl/g und einen Schmelzpunkt von 206,5 °C. Im DSC ist ein einzelner Glasübergang von 11,2 °C detektierbar.

### Beispiel 14

### In-situ-Polymerisation eines Triblockterpolymers der Zusammensetzung G/TMC/CL = 70/15/15 mit 50 Gew.-% eines Weichsegments der Zusammensetzung G/TMC/CL = 40/30/30

In der ersten Stufe werden 400,0 g Glykolid, 300,0 g 1,3-Dioxan-2-on und 300,0 g Caprolacton zusammen mit 1000 mg Diethylenglykol und 200 mg Zinnoctoat (Lösung in Diethylether) in analoger Reaktionsführung zu Beispiel 10 polymerisiert und in der 2. Stufe mit 1000,0 g Glykolid zum Triblockterpolymer umgesetzt.

Die inhärente Viskosität des Weichsegments vor der Glykolidzugabe beträgt 1,083 dl/g, der Glaspunkt liegt bei -15,6 °C. Das Triblockterpolymer besitzt eine inhärente Viskosität von 1,060 dl/g und einen Schmelzpunkt von 186,3 °C. Im DSC ist ein einzelner Glasübergang von 5,1 °C detektierbar.

### Verarbeitung der Polymere aus Beispiel 11 bis 14 zu Monofilamenten und deren Eigenschaften

Die Polymere aus den Beispielen 11 bis 14 wurden in ähnlicher Weise wie in Beispiel 9 und 10 zu Monofilamenten extrudiert, verstreckt und nachbehandelt (posttreated). Tabelle 2 zeigt die mechanischen Eigenschaften dieser Fasern in Abhängigkeit von der Zusammensetzung des Weichsegments und dessen Anteil im Triblockterpolymer.

**Tabelle 2**

| Einfluß der Kompatibilität zwischen Hart- und Weichsegment sowie Einfluß des Weichsegmentanteils auf die mechanischen Fasereigenschaften. Vergleich mit kommerziellen Produkten und Patenten. | | | | | | | | |
|---|---|---|---|---|---|---|---|---|
| Beispiel | Polym. Beisp. | WS-Anteil (Gew.-%) | Glykolid in WS (Gew.-%) | d (mm) | LRK (N/mm²) | Dehnung (%) | Modul (N/mm²) | KRK (N/mm²) |
| 11a Vergleichs beispiel | 11 | 38 | 26 | 0,500 | 476 | 38,7 | 2293 | 311 |
| 12a | 12 | 40 | 30 | 0,441 | 461 | 46,2 | 2012 | 348 |
| 13a | 13 | 43 | 35 | 0,475 | 595 | 37 | 1284 | 421 |
| 13b | 13 | 43 | 35 | 0,211 | 660 | 36,4 | 1335 | 592 |
| 13c | 13 | 43 | 35 | 0,360 | 642 | 41,7 | 1329 | 504 |
| 13d | 13 | 43 | 35 | 0,262 | 542 | 36,9 | 902 | 433 |
| 14a | 14 | 50 | 40 | 0,359 | 410 | 28,3 | 775 | 328 |
| USSC | US-Pat. | 35 | 0 | 0,301 | 383 | 21 | -- | 317 |
| Monocryl | USP 0 | | | 0,433 | 666 | 48,1 | 893 | 360 |
| Biosyn | USP 0 | | | 0,449 | 791 | 37,0 | 1530 | 405 |
| WS = Weichsegment d = Fadendurchmesser LRK = Linearreißkraft KRK = Knotenreißkraft -- = keine Angabe USSC = US-Patent Nr. 5431679 | | | | | | | | |

**Tabelle 3**

| Einfluß des Weichsegmentanteils und des Gesamtglykolidgehaltes auf die in vitro-Degradation im Sörensen-Puffer (pH 7,4 bei 37 °C). | | | | |
|---|---|---|---|---|
| Beispiel | WS-Anteil (Gew.-%) | Glykolid (gesamt) (Gew.-%) | KRK-Retention nach 7 Tagen (%) | KRK-Retention nach 14 Tagen (%) |
| 12a | 40 | 72 | 68,8 | 32,1 |
| 13a | 43 | 72 | 66,6 | 31,3 |
| 14a | 50 | 70 | 44,2 | 7,4 |
| 10 Vergleidesbeistriel | 54 | 73 | 41, 5 | 5, 6 |
| Monocryl | | | | |
| USP 0 | | | 40,0 | 15,0 |
| Biosyn | | | | |
| USP 0 | | | 64,8 | 44,1 |

Auffällig sind beim Faden 11a die mäßigen Festigkeiten, wobei hier der Hartsegmentanteil und damit die Kristallinität am größten ist.

Die Inkompatibilität zwischen Hart- und Weichsegment wirkt sich demnach negativ auf die mechanischen Eigenschaften aus. Darüber hinaus zeigt der Vergleich mit dem Faden aus dem Patent US 5431679, dessen Hartsegmente ebenfalls aus Glykolid, das Weichsegment jedoch nur aus Trimethylencarbonat und Caprolacton besteht, daß in der vorliegenden Erfindung durch das Einpolymerisieren von Glykolid in das Weichsegment die Verträglichkeit zum Hartsegment erhöht und die mechanischen Eigenschaften deutlich verbessert werden. Ferner wird ersichtlich, daß ein ausreichend niederer Modul - er ist ein Maß für die Biegeschlaffheit und damit die Flexibilität des Fadens - erst bei einem Weichsegmentanteil von 43 Gewichtsprozent oder mehr erzielt wird. Allerdings nehmen die Reißkräfte bei einer weiteren Erhöhung des Weichsegmentanteils ab, so daß die Polymerzusammensetzung der Fäden 13a bis 13d das Optimum darstellt.

Beim Vergleich der Fäden 12a und 13a bezüglich des in vitro-Degradationsverhaltens wird deutlich, daß sich zwischen einem Weichsegmentanteil von 40 und 43 Gewichtsprozent bei gleichem Gesamtglykolidanteil praktisch keine Unterschiede zeigen.

Da Glykolid unter den eingesetzten Monomeren das schnellstdegradierbare Polymer ergibt, ist die Degradationskinetik des Fadens 14a mit einem Gesamtglykolidgehalt von nur 70 Gew.-% überraschend. Hier scheint sich die verminderte Kristallinität aufgrund der Erhöhung des Weichsegmentanteils in einer Beschleunigung der Degradation zu äußern, was wohl auch für Beispiel 10 zutrifft.

Es ist festzustellen, daß mittels der beanspruchten Polymerkompositionen über eine Variation a) des Gesamtglykolidgehaltes und/oder b) des Weichsegmentanteils die Degradationskinetik innerhalb gewisser Grenzen variabel einstellbar ist.

Vergleicht man die Degradationskinetik der vorliegenden Erfindung mit den Werten der kommerziellen Produkte Monocryl (Fa. Eticon) und Biosyn (Fa. USSC), so ist ersichtlich, daß damit der Bereich zwischen diesen abgedeckt werden kann. Die gegenüber Monocryl verlängerte Degradationszeit in der bevorzugten Form der Erfindung ist besonders dann von Vorteil, wenn ein verzögerter Wundheilprozeß auftritt.

## Patentansprüche

1. Chirurgisches Nahtmaterial ganz oder teilweise gebildet aus einem Triblockterpolymer mit einer Struktur ABA gebildet aus einem biologisch abbaubaren Hartsegment A und einem biologisch abbaubaren Weichsegment B, worin das Weichsegment B dihydroxyterminiert und an die beiden Hartsegmente A chemisch gebunden ist, **dadurch gekennzeichnet, daß** die Hartsegmentblöcke 50 bis 60 Gew.-% des Triblockterpolymers umfassen, das Weichsegment ein statistisches Terpolymer gebildet aus Trimethylencarbonat, ε-Caprolacton und Glykolid mit völlig amorpher Struktur ist, im Terpolymer des Weichsegments B Trimethylencarbonat in einem Anteil von 5 bis 70 Gew.-%, ε-Caprolacton in einem Anteil von 5 bis 70 Gew.-% und Glykolid in einem Anteil von 10 bis 70 Gew.-% enthalten ist, Trimethylencarbonat und ε-Caprolacton in einem Gewichtsverhältnis zwischen 80 : 20 und 20 : 80 vorhanden sind, und Glykolid als Monomer sowohl im Hartsegment A wie im Weichsegment B vorhanden ist.

2. Nahtmaterial nach Anspruch 1, **dadurch gekennzeichnet, daß** das Terpolymer des Weichsegments B durch statistische Copolymerisation von Trimethylencarbonat, ε-Caprolacton und Glykolid hergestellt ist.

3. Verwendung eines Triblockterpolymers mit einer Struktur ABA gebildet aus einem biologisch abbaubaren Hartsegment A und einem biologisch abbaubaren Weichsegment B, worin das Weichsegment dihydroxyterminiert und an die beiden Hartsegmente A chemisch gebunden ist und die Hartsegmentblöcke 50 bis 60 Gew.-% des Triblockterpolymers umfassen, das Weichsegment ein statistisches Terpolymer gebildet aus Trimethylencarbonat, ε-Caprolacton und Glykolid mit völlig amorpher Struktur ist, im Terpolymer des Weichsegments B Trimethylencarbonat in einem Anteil von 5 bis 70 Gew.-%, ε-Caprolacton in einem Anteil von 5 bis 70 Gew.-% und Glykolid in einem Anteil von 10 bis 70 Gew.-% enthalten ist, Trimethylencarbonat und ε-Caprolacton in einem Gewichtsverhältnis zwischen 80 : 20 und 20 : 80 vorhanden sind, und Glykolid als Monomer sowohl im Hartsegment A wie im Weichsegment B vorhanden ist, insbesondere nach einem der vorhergehenden Ansprüche, zur Herstellung eines resorbierbaren Nahtmaterials für die Chirurgie.

4. Verwendung des Triblockterpolymers nach Anspruch 3 als chirurgischer Nähfaden in Form eines Monofilaments.

5. Verwendung des Triblockterpolymers nach Anspruch 3 als chirurgischer Nähfaden in Form eines multifilamenten Fadens.

6. Verfahren zur Herstellung eines chirurgischen Nahtmaterials ganz oder teilweise gebildet aus einem Triblockterpolymer mit einer Struktur ABA gebildet aus einem biologisch abbaubaren Hartsegment A und einem biologisch abbaubaren Weichsegment B, worin das Weichsegment dihydroxyterminiert ist und an die beiden Hartsegmente A chemisch gebunden wird, **dadurch gekennzeichnet, daß** das Triblockterpolymer durch chemisches Umsetzen des Hartsegmentmonomers mit Hydroxyendgruppen des Weichsegments B, das ein statistisches Terpolymer aus Trimethylencarbonat, ε-Caprolacton und Glykolid mit völlig amorpher Struktur ist, gebildet wird, so dass ein Triblockterpolymer mit einem Hartsegmentanteil von 50 bis 60 Gew.-% ausgebildet wird, wobei zuvor das Weichsegment durch statistische Copolymerisation von Trimethylencarbonat, ε-Caprolacton und Glykolid, bei einem Gewichtsanteil von Trimethylencarbonat von 5 bis 70 Gew.-%, bevorzugt 10 bis 40 %, ε-Caprolacton von 5 bis 70 Gew.-%, bevorzugt 10 bis 40 %, und Glykolid von 10 bis 70 Gew.-%, bevorzugt 30 bis 60 %, hergestellt und zu Filamenten versponnen wird.

7. Verfahren nach Anspruch 6, **dadurch gekennzeichnet, daß** das Nahtmaterial mit γ-Strahlen behandelt wird.

## Claims

1. Surgical suture material that is partially, or entirely, formed from a triblock terpolymer having a structure ABA formed from a biodegradable hard segment, A, and a biodegradable soft segment, B, where the soft segment, B, is dihydroxyl-terminated and chemically bonded to both hard segments, A, wherein the hard-segment blocks constitute 50 % to 60 % of the triblock terpolymer by weight, the soft segment is a random terpolymer formed from trimethylene carbonate, ε-caprolactone, and glycolide that has a totally amorphous structure, trimethylene carbonate is contained in the terpolymer of the soft segment, B, in a proportion ranging from 5 % to 70 % by weight, ε-caprolactone is contained therein in a proportion ranging from 5 % to 70 % by weight, and glycolide is contained therein in a proportion ranging from 10 % to 70 % by weight, trimethylene carbonate and ε-caprolactone are present in a proportion ranging from 80:20 to 20:80 by weight, and glycolide is present as a monomer in both the hard segment, A, and soft segment, B.

2. Suture material according to claim 1, wherein the terpolymer of the soft segment, B, is produced by random copolymerization of trimethylene carbonate, ε-caprolactone, and glycolide.

3. Employment of a triblock terpolymer having a structure ABA formed from a biodegradable hard segment, A, and a biodegradable soft segment, B, wherein the soft segment, B, is dihydroxyl-terminated and chemically bonded to both hard segments, A, and the hard-segment blocks constitute 50 % to 60 % of the triblock terpolymer by weight, the soft segment forms a random terpolymer formed from trimethylene carbonate, ε-caprolactone, and glycolide having a totally amorphous structure, trimethylene carbonate is contained in the terpolymer of the soft segment, B, in a proportion ranging from 5 % to 70 % by weight, ε-caprolactone is contained therein in a proportion ranging from 5 % to 70 % by weight, and glycolide is contained therein in a proportion ranging from 10 % to 70 % by weight, trimethylene carbonate and ε-caprolactone are present in a proportion ranging from 80:20 to 20:80 by weight, and glycolide is present as a monomer in both the hard segment, A, and soft segment, B, in particular, a triblock terpolymer according to any of the foregoing claims, as a material for manufacturing resorbable surgical sutures.

4. Employment of the triblock terpolymer according to claim 3 as a surgical suture in the form of a monofilament fiber.

5. Employment of the triblock terpolymer according to claim 3 as a surgical suture in the form of a multifilament fiber.

6. A process for producing a a surgical suture material that is partially, or entirely, formed from a triblock terpolymer having a structure ABA formed from a biodegradable hard segment, A, and a biodegradable soft segment, B, where the soft segment, B, is dihydroxyl-terminated and chemically bonded to both hard segments, A, wherein the triblock terpolymer is formed by chemically reacting the hydroxyl end groups of the soft segment, B and the hard-segment monomer such that a random terpolymer composed of trimethylene carbonate, ε-caprolactone, and glycolide having a totally amorphous structure will be formed in order that a triblock terpolymer having a hard-segment proportion ranging from 50 % to 60 % by weight will be configured, where the soft segment has been previously produced by random copolymerization of trimethylene carbonate, ε-caprolactone, and glycolide for a trimethylene-carbonate proportion ranging from 5 % to 70 %, and preferably ranging from 10 % to 40 %, by weight, an ε-caprolactone proportion ranging from 5 % to 70 %, and preferably ranging from 10 % to 40 %, by weight, and a glycolide proportion ranging from 10 % to 70 %, and preferably ranging from 30 % to 60 %, by weight, and spun into filaments.

7. A process according to claim 6, wherein the surgical suture material is treated with γ-radiation.

## Revendications

1. Matériel de suture chirurgical formé en tout ou partie d'un tri-terpolymère à blocs à structure ABA formé d'un segment dur A biodégradable et d'un segment mou B biodégradable, dans lequel le segment mou B possède un groupe dihydroxyle terminal et est relié chimiquement aux deux segments durs A, **caractérisé par le fait que** les blocs de segments durs représentent 50 à 60 % du pourcentage massique du tri-terpolymère à blocs, que le segment mou est un terpolymère statistique composé de carbonate de triméthylène, de caprolactone-ε et de glycolide à structure totalement amorphe, que le terpolymère du segment mou B est composé de carbonate de triméthylène à hauteur de 5 à 70 % du pourcentage massique, de caprolactone-ε à hauteur de 5 à 70 % du pourcentage massique, de glycolide à hauteur de 10 à 70 % du pourcentage massique, que le carbonate de trimethylène et le caprolactone-ε sont présents dans un rapport massique compris entre 80 : 20 et 20 : 80 et que le glycolide est présent sous forme de monomère aussi bien dans le segment dur A que dans le segment mou B.

2. Matériel de suture selon la revendication 1, **caractérisé par le fait que** le terpolymère du segment mou B est obtenu par copolymérisation statistique de carbonate de triméthylène, de caprolactone-ε et de glycolide.

3. Utilisation d'un tri-terpolymère à blocs à structure ABA formé d'un segment dur A biodégradable et d'un segment mou B biodégradable, dans lequel le segment mou possède un groupe dihydroxyle terminal et est relié chimiquement aux deux segments durs A, les blocs de segments durs représentant 50 à 60 % du pourcentage massique du tri-terpolymère à blocs, le segment mou étant un terpolymère statistique composé de carbonate de triméthylène, de caprolactone-ε et de glycolide à structure totalement amorphe, du carbonate de triméthyléne étant contenu dans le terpolymère du segment mou B à hauteur de 5 à 70 % du pourcentage massique, du caprolactone-ε à hauteur de 5 à 70 % du pourcentage massique et du glycolide à hauteur de 10 à 70 % du pourcentage massique, le carbonate de triméthylène et le caprolactone-ε étant présents dans un rapport massique compris entre 80 : 20 et 20 : 80, et le glycolide étant présent sous forme de monomère aussi bien dans le segment dur A que dans le segment mou B, notamment selon l'une des revendications précédentes, dans le but de fabriquer un matériel de suture résorbable pour des applications chirurgicales.

4. Utilisation du tri-terpolymère à blocs selon la revendication 3 comme fil de suture chirurgical sous forme de monofilament.

5. Utilisation du tri-terpolymère à blocs selon la revendication 3 comme fil de suture chirurgical sous forme de fil multifilament.

6. Procédé visant à fabriquer un matériel de suture chirurgical formé en tout ou partie d'un tri-terpolymère à blocs à structure ABA formé d'un segment dur A biodégradable et d'un segment mou B biodégradable, dans lequel le segment mou possède un groupe dihydroxyle terminal et est relié chimiquement aux deux segments durs A, **caractérisé par le fait que** le tri-terpolymère à blocs est formé par transformation chimique du monomère du segment dur avec les groupes dihydroxyles terminales du segment mou B, lequel étant un terpolymère statistique composé de carbonate de triméthylène, de caprolactone-ε et de glycolide à structure totalement amorphe, de sorte qu'un tri-terpolymère à blocs comprenant des segments durs représentant 50 à 60 % du pourcentage massique se forme, le segment mou étant tout d'abord obtenu par copolymérisation statistique de carbonate de triméthymène, de caprolatone-ε et de glycolide, pour un pourcentage massique atteignant 5 à 70 % pour le carbonate de triméthylène, de préférence de 10 à 40 %, 5 à 70 % pour le caprolactone-ε, de préférence de 10 à 40 %, 10 à 70 % pour le glycolide, de préférence de 30 à 60 %, et filé sous forme de filaments.

7. Procédé selon la revendication 6, **caractérisé par le fait que** le matériel de suture est traité aux rayons γ.
